# FASCICULE DE BREVET EUROPEEN

(11) **EP 2 027 097 B1**
(45) Date de publication et mention de la délivrance du brevet: **18.02.2015**
(21) Numéro de dépôt: 07766115.5
(22) Date de dépôt: 29.05.2007
(51) Int. Cl.: C07D 233/96, C07D 235/10

(54) **MONOMÈRES ET POLYMÈRES AMÉLIORÉS PORTEURS DE GROUPEMENTS IMIDAZOLES ET BENZIMIDAZOLES ET MEMBRANE À CONDUCTION PROTONIQUE EN CONTENANT POUR LA FABRICATION D'UNE PILE À COMBUSTIBLE**
VERBESSERTE MONOMERE UND POLYMERE MIT IMIDAZOL- UND BENZIMIDAZOLGRUPPIERUNGEN SOWIE DIESE ENTHALTENDE PROTONENAUSTAUSCHMEMBRAN ZUR HERSTELLUNG EINER BRENNSTOFFZELLE
IMPROVED MONOMERS AND POLYMERS CARRYING IMIDAZOLE AND BENZIMIDAZOLE GROUPINGS, AND PROTON EXCHANGE MEMBRANE CONTAINING THE SAME FOR THE PRODUCTION OF A FUEL CELL

(30) Priorité: 07.06.2006 FR 0652047; 07.06.2006 FR 0652048
(43) Date de publication de la demande: 25.02.2009
(73) Titulaire: Peugeot Citroën Automobiles SA, 78140 Vélizy Villacoublay (FR); Centre National de la Recherche Scientifique (CNRS), 75016 Paris (FR)
(72) Inventeur: GLIPA, Xavier, F-78480 Verneuil Sur Seine (FR); AMEDURI, Bruno, F-34000 Montpellier (FR); DELON, Louis, F-34980 St Gely Du Fesc (FR); JONES, Deborah, F-34380 St Martin de Londres (FR); ROZIERE, Jacques, F-34380 Martin De Londres (FR); FRUTSAERT, Guillaume, F-34090 Montpellier (FR)
(74) Mandataire: Renous Chan, Véronique
(86) Numéro de dépôt international: PCT/FR2007/051348
(87) Numéro de publication internationale: WO 2007/141441

(56) Documents cités:
- FR-A- 2 877 147
- JP-A- 62 116 564
- US-B2- 6 750 352
- DOMINA ET AL: IZVESTIA AKADEMII NAUK SSSR. SERIA HIMICESKAA, MOSCOW, RU, vol. 9, 1979, pages 2096-2102, XP009078697 ISSN: 0002-3353
- BAIKALOVA ET AL: IZVESTIA AKADEMII NAUK SSSR. SERIA HIMICESKAA, MOSCOW, RU, vol. 5, 1977, pages 1158-1161, XP009078698 ISSN: 0002-3353
- SAVADOGO O: "Emerging membranes for electrochemical systems - Part II. High temperature composite membranes for polymer electrolyte fuel cell (PEFC) applications" JOURNAL OF POWER SOURCES, ELSEVIER, AMSTERDAM, NL, vol. 127, no. 1-2, 10 mars 2004 (2004-03-10), pages 135-161, XP004494974 ISSN: 0378-7753
- TROITSKAYA ET AL.: ZHURNAL ORGANICHESKOI KHIMII, vol. 10, 1974, pages 1524-1529, XP009078686

## Description

La présente invention concerne principalement des monomères et polymères pouvant être utilisés pour la fabrication d'une membrane à conduction protonique en l'absence d'eau.

L'invention concerne également un procédé de fabrication de ces monomères et polymères, les membranes pouvant être fabriquées à partir de ces monomères, ainsi que l'utilisation de ces membranes comme électrolyte solide polymère ou comme membrane échangeuse de protons au sein d'une pile à combustible.

Dans une telle pile, un électrolyte est pris « en sandwich » entre deux couches actives qui sont le siège des réactions anodiques et cathodiques assurant la conversion de l'énergie chimique en énergie électrique.

Selon cette configuration, l'électrolyte doit assurer le transfert protonique d'une couche active vers l'autre.

A cet effet, les piles à combustible sont à électrolyte solide polymère (SPEFC pour Solid Polymer Electrolyte Fuel Cell) ou à membrane échangeuse de protons (Proton Exchange Membrane Fuel Cell) et sont certainement les plus élégantes en terme de conception et de fonctionnement.

Pour assurer le transport des protons, les membranes connues contiennent 20 à 30% d'eau, les molécules d'eau assurant la solvatation des protons qui deviennent alors extrêmement mobiles au sein de la membrane.

Lorsque le polymère constituant la membrane comporte en outre des fonctions acides, les molécules d'eau permettent la dissociation des groupes acides et de ces fonctions, libérant ainsi les protons dont elles assurent ensuite le transport.

Les performances des membranes sont en conséquence limitées par la quantité d'eau qu'elles contiennent, la déshydratation possible de la membrane pouvant amener à l'interruption du transport protonique.

Cette déshydratation peut survenir en raison de la mobilité des molécules d'eau et/ou en raison de la température de fonctionnement de la pile.

En effet, les molécules d'eau, non liées à la structure polymère de la membrane, sont mobiles et peuvent être entraînées notamment par des flux importants de protons à travers la membrane, amenuisant par conséquent ses propriétés conductrices, jusqu'à l'interruption totale du transport protonique.

Seules des procédures additionnelles permettraient d'éviter la déshydratation due à la mobilité des molécules d'eau, mais celles-ci apportent en contre partie un surcoût et une complication du système pile (auxiliaires dédiés à l'humidification).

D'autre part, il existe une température limite de fonctionnement des piles correspondant naturellement à la température d'ébullition de l'eau de 100°C à pression atmosphérique.

Cette température constitue une limite physique, au-delà de laquelle la quantité d'eau diminue de façon considérable, jusqu'à l'assèchement complet de la membrane.

Une solution toute indiquée par les lois de thermodynamique consisterait à augmenter la pression de fonctionnement du système, la température d'ébullition de l'eau augmentant en conséquence.

Cependant, cette solution n'est pas satisfaisante car pour des pressions de fonctionnement importantes, le flux de protons traversant la membrane entraîne d'autant plus de molécules d'eau, pénalisant au final les performances de la pile.

La plupart des polymères constituant les membranes ionomères utilisées dans les piles à combustibles comportent des groupements protoniques SO₃H, PO₃H₂. Ces groupements fonctionnels sont dissociés en présence d'eau, ou en présence de molécules ou de groupements fonctionnels basiques. Lorsque ces groupements basiques présentent différents sites donneurs et accepteurs de protons, ils peuvent jouer un rôle identique à celui des molécules d'eau dans le transport protonique. Cependant, dans ce type de membranes, des problèmes d'élution peuvent survenir lors du fonctionnement de la pile à combustible.

Dans ce contexte, l'invention vise principalement à remédier aux inconvénients précités par la mise au point d'une membrane assurant le transport protonique à des températures de fonctionnement supérieures à 100°C et dont le procédé de fabrication présente un rendement important.

A cet effet, la membrane selon l'invention est faite à base d'un polymère hétérocyclique comprenant des hétérocycles qui assurent le transport des protons au sein de la membrane y compris en l'absence d'eau. Ainsi, la migration des protons est assurée par le biais des hétérocycles qui offrent un avantage supplémentaire par rapport aux molécules d'eau, étant donné que ces hétérocycles sont ancrés au sein de la membrane, évitant tout problème d'élution.

Plus précisément, l'invention concerne un monomère porteur d'un hétérocycle de type imidazole, pouvant être utilisé pour la fabrication d'une membrane conductrice ionique, et dont la structure chimique comprend au moins un motif de formule : 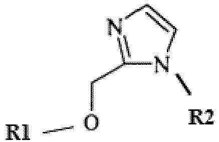 dans laquelle R1 comprend un groupement alkenyl, et dans laquelle R2 comprend un groupement de protection de l'un des atomes d'azote de l'hétérocycle choisi parmi_un groupement benzyle ou trialkylsilyl.

Selon une variante de réalisation, R1 est de formule chimique CH=CH₂.

Selon une autre variante de réalisation, R1 est de formule chimique (CH₂)₂-O-CH=CH₂.

De plus, le procédé de fabrication du polymère constitutif de la membrane selon l'invention consiste à fabriquer un monomère pourvu d'un hétérocycle de type imidazole et d'un groupement de protection de l'un des atomes d'azote de cet hétérocycle, à polymériser ce monomère protégé pour obtenir un polymère protégé, à faire subir au polymère protégé une étape de déprotection des atomes d'azote des hétérocycles, par laquelle est obtenu un polymère conducteur protonique, et présente un rendement important.

L'invention concerne également un monomère de type benzimidazole, pouvant être utilisé pour la fabrication d'une membrane conductrice ionique.

Selon l'invention, la structure chimique de ce monomère comprend au moins un motif de formule : 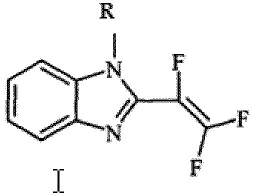 dans laquelle R est un groupement benzyl ou trialkylsilyl de protection de l'un des atomes d'azote de l'hétérocycle.

De plus, le procédé de fabrication du polymère constitutif de la membrane selon l'invention consiste à fabriquer un monomère pourvu d'un hétérocycle de type benzimidazole et d'un groupement de protection de l'un des atomes d'azote de l' hétérocycle, à polymériser ce monomère protégé pour obtenir un polymère protégé, à faire subir au polymère protégé une étape de déprotection des atomes d'azote des hétérocycles par laquelle est obtenu un polymère conducteur protonique, et présente un rendement important.

Ce procédé comprend : au moins une étape de réaction entre un composé comprenant une ortho-phénylène diamine, et un monomère fluoré, en présence d'un solvant organique, par laquelle est obtenue une molécule intermédiaire fluorée et porteuse d'un hétérocycle de type benzimidazole,
- au moins une étape de protection d'un atome d'azote du groupement benzimidazole de la molécule intermédiaire par un groupement R tel qu'un groupement benzyl ou un groupement trialkylsilyl (RIR2R3Si) ou un groupement carbamate (CO[2]RI), - au moins une étape de déshydrofluoration du composé obtenu à l'étape par réaction de ce composé avec une base.

Selon une autre caractéristique, l'invention concerne un polymère protégé comprenant comme motif le monomère de type benzimidazole selon l'invention.

L'invention concerne encore le polymère déprotégé fait à base du polymère protégé, et comprenant des atomes d'hydrogène en remplacement des groupements de protection (R).

L'invention concerne également la membrane conductrice ionique, faite à base du polymère déprotégé, la pile à combustible impliquant comme électrolyte cette membrane conductrice ionique et l'électrolyseur comprenant une cellule impliquant comme électrolyte cette même membrane.

L'invention sera mieux comprise et d'autres buts, avantages et caractéristiques de celle-ci apparaîtront clairement à la lecture de la description qui suit, faite en référence aux figures annexées parmi lesquelles :
• la figure 1 représente des exemples d'hétérocycles pouvant être intégrés au polymère selon 1 ' invention ;
• la figure 2 illustre la synthèse d'un premier monomère présentant un hétérocycle de type imidazole selon l'invention ;
• la figure 3 montre la synthèse d'une molécule intermédiaire intervenant comme réactif dans la réaction de la figure 2 ;
• la figure 4 représente la synthèse d'un polymère selon l'invention à partir du premier monomère de la figure 2;
• la figure 5 illustre la caractérisation RMN du ¹⁹F du polymère de la figure 4;
• la figure 6 représente la caractérisation RMN du ¹H du polymère de la figure 4 ;
- la figure 7 illustre la formation d'un second polymère à imidazole ;
- la figure 8 montre la formation d'un second monomère à hétérocycle de type imidazole ;
- la figure 9 est une illustration du 1-benzimidazole 9 de la figure 3 ;
- la figure 10 représente la molécule intermédiaire 7 de la figure 3 ;
- la figure 11 illustre le spectre RMN ¹H obtenu pour la molécule intermédiaire 7 ;
- la figure 12 est une illustration du premier monomère selon l'invention ;
- la figure 13 représente le spectre RMN ¹H obtenu pour le monomère de la figure 12 ;
- la figure 14 illustre une réaction connue de l'homme du métier, de formation d'un monomère fluoré à benzimidazole ;
la figure 15 illustre la première étape (a) de formation d'un premier monomère protégé selon l'invention ;
- la figure 16 montre la deuxième (b) et la troisième (c) étape du procédé de fabrication du premier monomère protégé de l'invention ;
- la figure 17 représente les spectres RMN ¹⁹F et ¹H obtenus pour le monomère formé par l'étape (c) de la figure 16 ;
- la figure 18 représente un deuxième monomère à benzimidazole ;
- la figure 19 illustre la première étape (a) d'un procédé de fabrication d'un deuxième monomère protégé selon l'invention ;
- la figure 20 montre la deuxième étape (b) du procédé de fabrication du deuxième monomère protégé ;
- la figure 21 représente la troisième étape (c) de fabrication du deuxième monomère protégé ;
- la figure 22 illustre la polymérisation (d) du monomère protégé de la figure 21 et la déprotection des atomes d'azote (e) du polymère protégé obtenu à l'étape (d).

La présente invention propose l'élaboration de polymères particuliers formant des membranes permettant de présenter des propriétés de conduction protonique même à des températures supérieures à 100°C, telles que 200°C, à pression atmosphérique.

La migration des protons est assurée par le biais d'hétérocycles 2, immobilisés et ancrés à la structure polymère de la membrane.

De cette façon, les hétérocycles 2 n'accompagnent pas le proton dans sa migration, même pour des flux de protons élevés, et maintiennent par conséquent la complète efficacité de la pile, y compris à haute température.

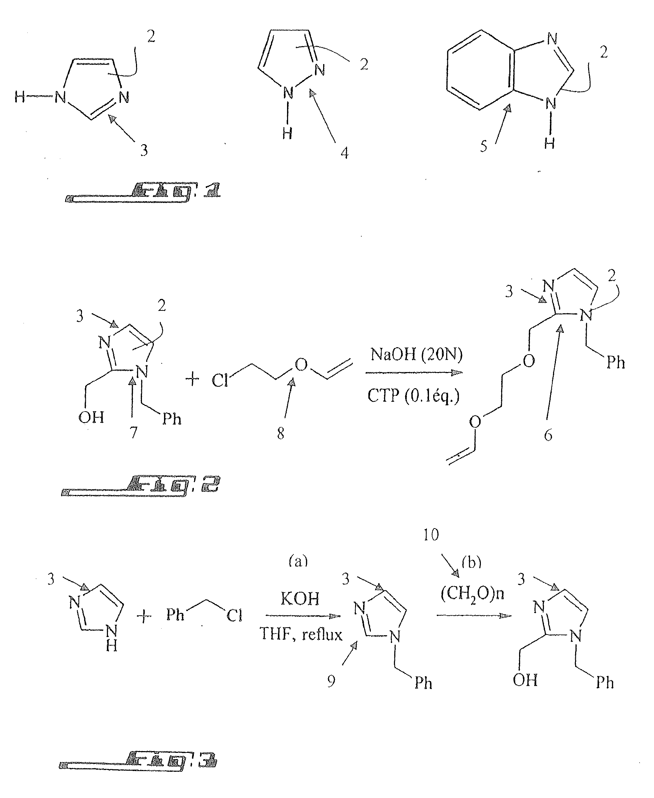

L'approche est ainsi basée sur l'utilisation d'hétérocycles 2 tels que des imidazoles 3, pyrazoles 4, benzimidazoles 5, illustrés sur la figure 1, comme solvants des protons, en remplacement des molécules d'eau des membranes connues.

En effet, tout comme l'eau, ces hétérocycles 2 forment des réseaux de liaisons hydrogène, et possèdent des propriétés de transport de protons similaires à celles de l'eau.

Dans un tel environnement d'hétérocycles immobilisés 2, le transport protonique repose essentiellement sur un mécanisme de diffusion connu, de type Grotthuss, impliquant un transfert du proton entre hétérocycles 2 et leur réorganisation par exemple par réorientation.

De nombreux types de polymères porteurs d'hétérocycles 2 peuvent être synthétisés et il est détaillé dans ce qui suit une famille de polymères comprenant des hétérocycles imidazoles 3 ainsi que différents procédés permettant leur fabrication.

Un premier procédé de fabrication de ce polymère consiste en l'homopolymérisation cationique de monomères à imidazole 6, tels que les 1-benzyl-2-(vinyloxyéthyloxyméthyl)imidazoles 6 en utilisant des amorceurs du type ZnI₂, HgI₂ ou tout autre complexe iodé et même des sels de Crivello.

La synthèse de ce monomère 6, représentée sur la figure 2, est effectuée en catalyse par transfert de phase par réaction d'une molécule intermédiaire 7, telle que le 1-benzyl-2-(hydroxyméthyl)imidazole 7, sur le 2-chloroéthyl vinyl éther 8.

Le catalyseur de transfert de phase utilisé dans cette réaction est le chlorure de triéthyl benzyl ammonium et les rendements obtenus varient entre 85 et 90%.

L'étape de formation de la molécule intermédiaire 7, connue de l'homme du métier, est représentée sur la figure 3, et consiste en une réaction entre le 1-benzylimidazole 9 et le formaldéhyde ou le paraformaldéhyde 10.

On obtient par l'homopolymérisation cationique susmentionnée du monomère 6, un polymère sur lequel sont ancrés des hétérocycles imidazoles 3, dont les atomes d'azote sont protégés par un groupement benzyl et peuvent être déprotégés par réaction du polymère avec le Pd(OH)₂.

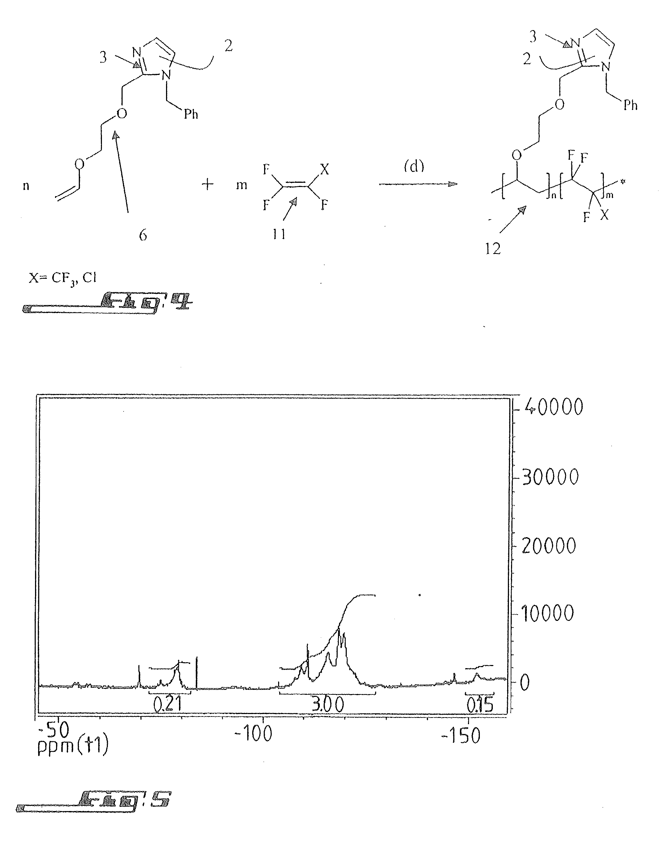

Un deuxième procédé de fabrication d'un polymère à imidazole 3 selon l'invention, consiste en une copolymérisation radicalaire représentée sur la figure 4 du monomère 6 décrit précédemment avec des monomères fluorés 11, tels que le tetrafluoroéthylène (TFE), l'hexafluoropropène (HFP, X=CF₃) et/ou le chlorotrifluoroéthylène (CTFE, X=Cl), ou le bromorotrifluoroéthylène (XTFE, X=Br), le trifluoroéthylène, le pentafluoropropene, le 3,3,3-trifluoropropene, le perfluoro methyl vinyl ether (PMVE), le perfluoro ethyl vinyl ether (PEVE), le perfluoro propyl vinyl ether (PPVE), ce dont il résulte un premier polymère fluoré 12 porteur de fonctions imidazoles 3.

La copolymérisation radicalaire est réalisée avec des amorceurs radicalaires de type peroxydes, azo, peroxycarbonates ou peroxypivalates dans une autoclave sous une pression comprise entre 5 et 50 bar, à une température fixée en fonction de la nature de l'amorceur utilisé, de sorte qu'à cette température l'amorceur ait une durée de demi-vie d'environ 1 heure.

Ce polymère 12 ainsi formé a notamment été caractérisé grâce à la spectroscopie Résonance Magnétique Nucléaire du ¹⁹F et du ¹H par une analyse du produit de la réaction de copolymérisation, après évaporation du solvant et purification par précipitation dans le pentane.

Le spectre RMN ¹⁹F obtenu (figure 5), confirme l'obtention du copolymère fluoré 12.

En effet, les signaux complexes visibles sur ce spectre, situés entre -70 et -85 ppm et entre -108 et -125 ppm, sont caractéristiques du déplacement chimique des atomes de fluor des motifs chlorotrifluoroéthylène (CTFE) dans le copolymère CTFE-éther vinylique.

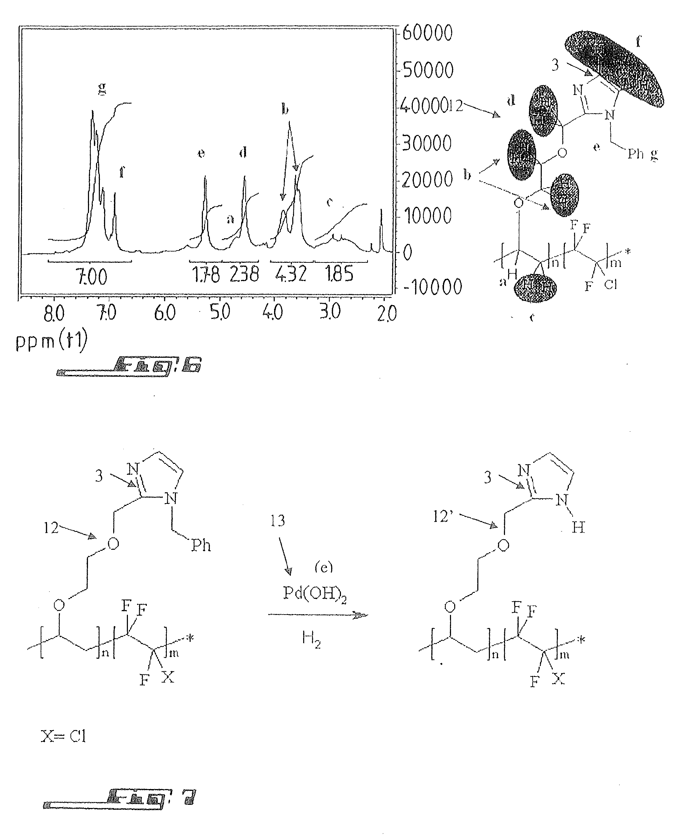

D'autre part, le spectre RMN ¹H confirme l'incorporation du monomère 6 dans le polymère 12 (figure 6) et l'absence de transfert au monomère, solvant, amorceur et polymère.

Le polymère 12 est ainsi constitué par une succession de monomères à imidazole 6 qui comportent chacun un groupement de protection de l'atome d'azote, à savoir un groupement benzyl.

On fait subir au polymère protégé obtenu 12 une étape de déprotection représentée sur la figure 7 au cours de laquelle les groupements de protection benzyl sont remplacés par des atomes d'hydrogène, de façon à former les groupements imidazoles qui assureront la conduction protonique.

Cette étape de déprotection consiste en une réaction du premier polymère 12 en présence de dihydrogène et de dihydroxyle de palladium Pd(OH)₂ 13.

Le polymère déprotégé 12' ainsi obtenu est semblable au premier polymère 12, mais comprend un atome d'hydrogène en remplacement du groupe benzyle du premier polymère 12, et présente en conséquence des propriétés conductrices protoniques.

A titre indicatif, on donne dans ce qui suit le détail des expériences menées en vue de la formation du polymère déprotégé 12' :

### Synthèse du 1-benzylimidazole 9 illustrée par l'étape (a) de la figure 3.

Selon le protocole expérimental retenu, 44 g d'imidazole (0,65 mol.), 100 g de chlorure de benzyle (0,78 mol.) et 60 g de KOH (1,07 mol.) dans 700 mL de THF sont placés dans un ballon de 1 L. Le mélange réactionnel est porté à reflux durant 120 h puis refroidi à température ambiante. La phase organique est filtrée et le solvant est évaporé sous pression réduite. Le produit solide obtenu est dissout dans 500 mL de CH2Cl2 puis lavé à l'eau (3 x 100 mL). La phase organique est séchée sur Na₂SO₄, filtrée puis évaporée sous pression réduite. Le solide recueilli est recristallisé dans le toluène pour donner 67 g de produit désiré.

### Synthèse du 1-benzyl-2-hydroxyméthyl-imidazole 7 représentée par l'étape (b) de la figure 3

50 g de 1-benzyl-imidazole du produit brut de la réaction précédente et 15 g de paraformaldéhyde sont dissoutes dans 150 mL de dioxane. Le mélange est porté à 135°C sous autoclave durant 20 h. Le solvant est évaporé sous pression réduite, le produit obtenu sous forme d'une huile noire est dissout dans 400 mL de CH₂Cl₂ puis lavé à l'eau (2x 100 mL). La phase organique est séchée sur Na₂SO₄, filtrée puis évaporée sous pression réduite. Le produit est tiré sous vide durant 24 h puis recristallisé dans 100 mL d'acétate d'éthyle pour donner 30 g de produit pur (rendement = 50% à partir de l'imidazole).

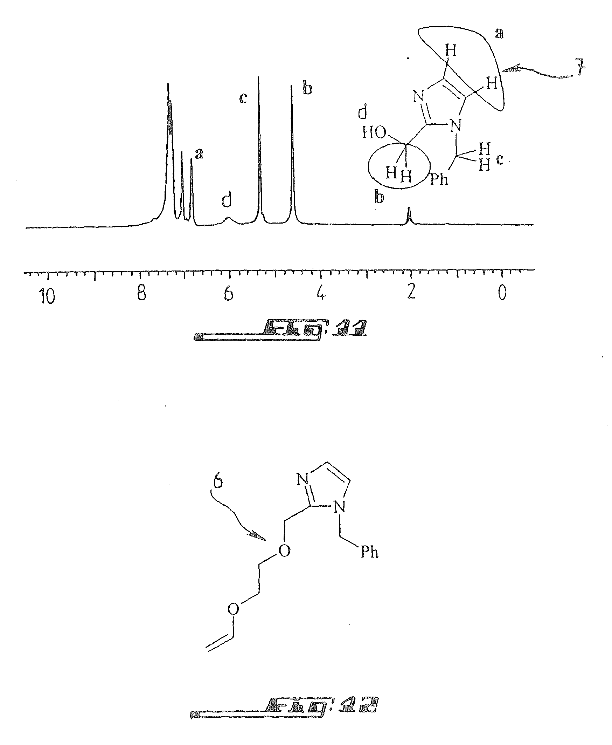

Le spectre RMN ¹H obtenu pour le produit final, visible sur la figure 11, confirme l'obtention du 1-benzyl-2-hydroxyméthyl-imidazole 7.

### Synthèse du 1-benzyl-2-(vinyloxyéthyloxyméthyl)imidazole 6 représentée par la figure 2

A une solution de 1-benzyl-2-hydroxyméthylimidazole (4,7 g, 0,024 mol.) dans le toluène (15 mL) sont ajoutés 0,54 g de TEBAC (2,4.10⁻³ mol) et 5,6 g de CEVE (0,05 mol). Une solution basique 20 N en NaOH (100 mL) est ensuite additionnée dans le milieu et le mélange hétérogène est porté à 90°C durant 24 h sous une agitation vigoureuse. Après refroidissement, la solution est diluée avec 250 mL d'éther décantée puis la phase organique est lavée à l'eau jusqu'à pH = 7 (10 x 100 mL). La phase organique est séchée sur Na₂SO₄, filtrée puis évaporée sous pression réduite pour donner 5,3 g (85%) de produit désiré. Etant donné la pureté satisfaisante du produit obtenu, aucune purification supplémentaire n'est requise.

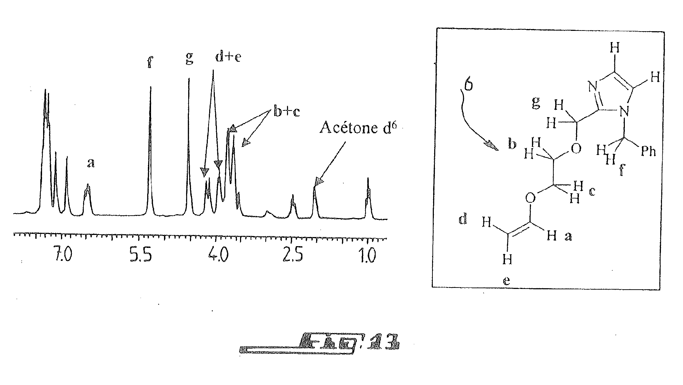

Le spectre RMN ¹H du produit obtenu, représenté sur la figure 13 confirme l'obtention du 1-benzyl-2-(vinyloxyéthyloxyméthyl)imidazole 6.

### Synthèse du copolymère poly(E.V-alt-CTFE) ou polymère protégé 12 illustrée par l'étape (d) de la figure 4

La copolymérisation du 1-benzyl-2 (vinyloxyéthyloxyméthyl) imidazole 6 avec le CTFE est effectuée dans une autoclave de 100 cm³ en hastelloy équipée d'un barreau magnétique, de deux vannes (entrée et sortie de gaz), d'un disque de sécurité, d'un manomètre de précision et d'un thermomètre.

L'autoclave est tout d'abord purgée de l'air qu'il contient grâce à un vide poussé. 10 g du monomère liquide (éther vinylique présentant le groupement imidazole protégé), 0,210 g d'amorceur tertiobutylpéroxypivalate et 60 mL de 1,1,1,3,3-pentafluorobutane sont introduits dans le système.

L'ensemble est ensuite refroidi dans un bain d'acétone à - 80°C et 10,1 g de CTFE sont introduits dans le système. L'autoclave est finalement placée dans un bain d'huile à 75°C pendant 14 heures, la pression atteint 15 bar en début de manipulation pour finir à 12 bar après 14 heures.

On laisse ensuite le système revenir à température ambiante, le CTFE n'ayant pas réagit est purgé et le brut réactionnel est évaporé pour obtenir une huile visqueuse noire. Le produit brun est solubilisé dans le dichlorométhane puis précipité dans le pentane pour obtenir une poudre de couleur orange.

Déprotection des imidazoles du copolymère poly(E.V-alt-CTFE) par hydrogénation visible sur la figure 7 (étape (e))

Dans la même autoclave que celle précédemment utilisée, on introduit 100 mL d'acide acétique, 4 g de copolymère poly(E.V-alt-CTFE) et 1 g de catalyseur de type dihydroxyde de palladium. L'autoclave est ensuite fermée et 32 bars d'hydrogène y sont introduits. Le mélange réactionnel est chauffé à 70°C pendant 48 heures. Le brut réactionnel est filtré sur célite puis le solvant est évaporé. Le produit obtenu est mis à reflux dans 50 ml d'une solution de Na₂CO₃ à 5%. Le produit est filtré, lavé avec trois fois 50ml d'eau distillée puis séché. Il est finalement repris dans le méthanol et précipité dans l'éther. 2 g de solide orange est obtenu avec un taux de déprotection de 90%.

Les analyses réalisées sur le produit final montrent que celui-ci présente une température de transition vitreuse de - 33°C et une stabilité thermique allant jusque 200°C pour une masse molaire moyenne de 8500 g.mol⁻¹ (équivalent PMMA).

Ces étapes permettent de former un polymère conducteur protonique présentant comme motif le monomère 6 de la figure 12.

Il est possible de préparer un polymère à groupements imidazole 3 selon un troisième procédé de fabrication consistant en une polymérisation d'un second monomère 14 à imidazole, le 1-benzyl-2-(vinyloxyméthyl)imidazole.

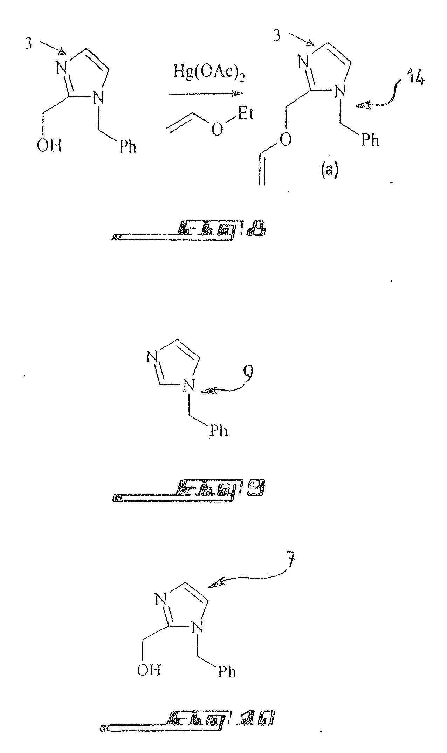

Comme visible sur la figure 8, la synthèse de ce monomère 14 consiste en une réaction de transéthérification de la molécule intermédiaire 7 de la figure 3 par l'éthyl vinyl éther 15, en présence de catalyseurs de transéthérification comme l'acétate de palladium Pd(OAc)₂ ou de mercure Hg(OAc)₂ 16.

Par la suite, ce monomère 14 est polymérisé, soit par homopolymérisation préalablement décrite en référence au premier procédé, soit par copolymérisation avec des monomères fluorés 11 comme décrit en référence au second procédé, ce dont il résulte la formation d'un polymère protégé à imidazole fluoré ou non.

Comme décrit pour les premier et deuxième procédés décrits ci-dessus, le polymère obtenu par le troisième procédé peut réagir avec le Pd(OH)₂ pour obtenir un polymère déprotégé, c'est-à-dire dépourvu des groupements benzyl et qui sera conducteur protonique.

Un quatrième procédé de formation du polymère selon l'invention peut consister en une terpolymérisation de l'un ou l'autre des monomères 6 et 14 ci-dessus décrits et illustrés respectivement sur les figures 2 et 8, avec deux alcènes fluorés, choisis parmi le tetrafluoroéthylène (TFE), l'hexafluoropropène (HFP, X=CF₃) et/ou le chlorotrifluoroéthylène (CTFE, X=Cl), ou le bromorotrifluoroéthylène (XTFE, X=Br), le trifluoroéthylène, le pentafluoropropene, le 3,3,3-trifluoropropene, le perfluoroalkyl methyl vinyl ether (PMVE), le perfluoroalkyl ethyl vinyl ether (PEVE), le perfluoroalkyl propyl vinyl ether (PPVE) ou tout autre molécule analogue présentant une plus longue chaîne fluorée.

Comme pour les polymères fabriqués par les procédés ci-dessus, le polymère obtenu par le quatrième procédé peut être déprotégé par réaction avec le Pd(OH)₂ et former un polymère conducteur protonique.

Les différents polymères déprotégés obtenus à partir des quatre procédés ci-dessus décrits peuvent constituer la base de membranes conductrices protoniques.

Il est à noter que différents monomères porteurs de fonction imidazole peuvent être fabriqués selon un procédé de fabrication similaire au premier procédé de fabrication du monomère 6, mais dans lequel le réactif chloroéthylvinyléther 8 serait remplacé par exemple par un composé de chaîne plus longue, comprenant un tel chloroéthylvinyléther 8.

De même, d'autres monomères selon l'invention peuvent être réalisés au moyen d'un procédé similaire au deuxième procédé de fabrication du monomère 14, dans lequel le réactif l'éthylvinyléther 15 serait remplacé notamment par un composé de chaîne plus longue et comprenant cet éthylvinyléther 15.

Ainsi, d'autres polymères que ceux décrits ci-dessus peuvent être fabriqués à partir de ces monomères et constituer différentes membranes conductrices ioniques, qui pourront servir d'électrolytes au sein de différents dispositifs éléctrochimiques tels qu'un électrolyseur ou une pile à combustible utilisée par exemple pour assurer la traction d'un véhicule automobile.

La membrane selon l'invention utilisée au sein d'une pile à combustible permet de dépasser la température limite de fonctionnement des piles à combustible à électrolyte solide polymère connues, de réduire la complexité du Système Pile (pile et ses auxiliaires), et réduire par conséquent son coût (les auxiliaires dédiés à humidification n'étant plus nécessaires).

Les hétérocycles des polymères déprotégés formés suivant les procédés de fabrication précités, assurent le transport protonique au sein de la membrane, alors que les groupements fluorés présents au sein du polymère pour certains procédés de fabrication (copolymérisation avec des alcènes fluorés, ou monomères porteurs d'hétérocycle fluoré) dotent le polymère d'une certaine inertie chimique en milieu oxydant ou réducteur et d'une thermostabilité qui participent à la stabilisation de la membrane en fonctionnement, notamment pour une utilisation au sein d'une pile à combustible opérant à des températures supérieures à 100°C.

D'autres types de polymères porteurs d'hétérocycles 2 peuvent être synthétisés et il est détaillé dans ce qui suit une famille de polymère selon l'invention comprenant des hétérocycles benzimidazoles 20.

La fabrication d'un polymère doté d'hétérocycles benzimidazoles 20, est obtenue à partir de la polymérisation de monomères à benzimidazole.

Cette polymérisation peut consister en une homopolymérisation radicalaire de monomères à benzimidazole, en utilisant des amorceurs radicalaire de type peroxydes, azo, peroxycarbonates ou peroxypivalates ou en une copolymérisation radicalaire de ce monomère avec des monomères fluorés, tels que le tetrafluoroéthylène, le trifluoroéthylene, l'hexafluoropropène (HFP, X=CF₃) et/ou le chlorotrifluoroéthylène (CTFE, X=Cl), le fluorure de vinyle, le 2H-1,1,3,3,3-pentafluoroéthylène, le fluorure de vinylidène, ou tout perfluoroalkylvinyléther dont la chaîne alkyle comprend entre 1 et 20 atomes de carbone.

On détaille dans ce qui suit différents procédés de fabrication de monomères à benzimidazole pouvant être utilisés pour la fabrication du polymère selon l'invention.

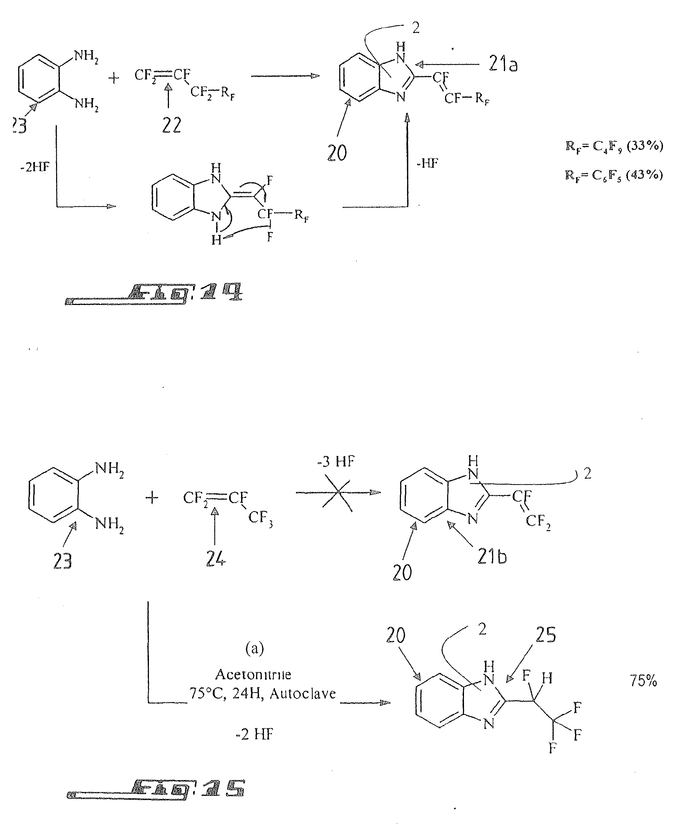

Comme illustré sur la figure 14, un tel monomère 21a peut être obtenu par un premier procédé connu de l'homme du métier entre une oléfine perfluorée 22 et une ortho phénylène diamine 23, ce monomère 21a comprenant un groupement R_{F} pouvant être un groupement C₄ F₉ ou C₆ F₅.

Les rendements de cette réaction sont cependant très faibles.

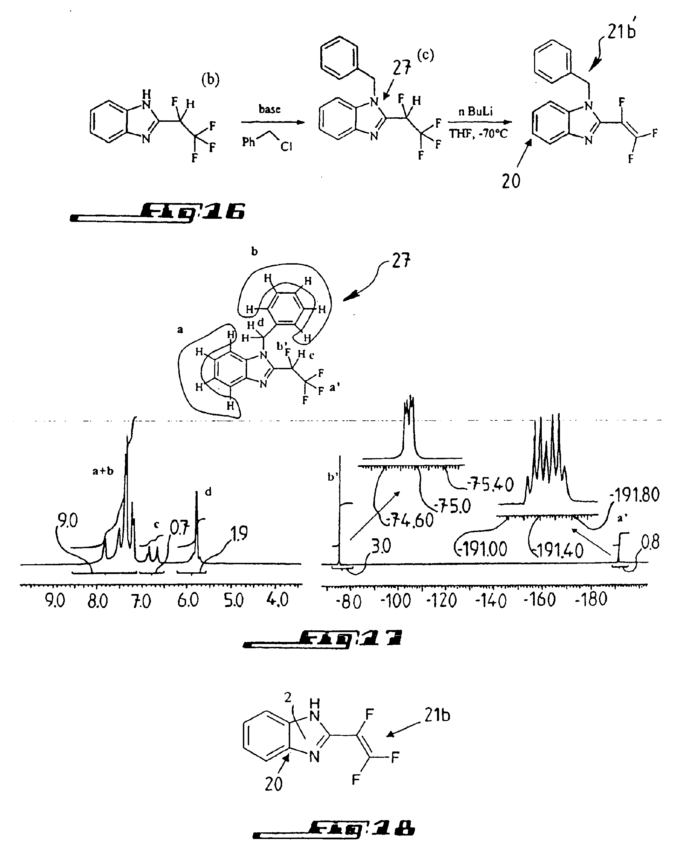

Un deuxième procédé de fabrication d'un monomère à benzimidazole dont le rendement de réaction est plus important consiste en une synthèse d'un deuxième monomère à benzimidazole 21b', semblable au monomère 21a précité mais étant dépourvu du groupement R_{F} de ce monomère et comprenant en outre, comme visible sur la figure 16, un élément de protection de l'un des atomes d'azote tel qu'un groupement benzyl greffé sur cet atome.

Ce deuxième monomère 21b' est obtenu en trois étapes illustrées respectivement aux figures 15 et 16.

La première étape (a) illustrée sur la figure 15, est une réaction entre l'orthophenylènediamine 23 et l'hexafluoropropène 24 sous autoclave durant 24 heures et le dérivé résultant est obtenu avec un bon rendement (75%). Cette réaction ne permet pas d'obtenir un monomère vinylique fluoré fonctionnalisé benzimidazole 21b, mais l'obtention du 2-(1,1,1,2- tetrafluoroéthyl) benzimidazole 10, après recristallisation dans un mélange eau / éthanol et avec un rendement de 75%.

La deuxième étape (b), détaillée sur la figure 16, consiste en une protection du groupement benzimidazole du 2-(1,1,1,2- tetrafluoroéthyl) benzimidazole 25 effectuée par réaction de ce composé 26 avec du chlorure de benzyle en présence d'une base, par laquelle est obtenu un produit intermédiaire 27 dans lequel l'atome d'hydrogène de l'un des atomes d'azote du groupement benzimidazole est remplacé par un groupement benzyl .

La troisième étape (c) est une déhydrofluoration de ce produit intermédiaire 27 au moyen du butyl lithium 13 en présence du THF (tetrahydrofurane) et à une température proche de -70°C, grâce à laquelle on obtient un monomère protégé 21b'.

On donne le détail expérimental des trois étapes (a), (b), (c) précitées :

### - Etape (a) : Synthèse du 2-(1,2,2,2-tetrafluoroéthyl)-benzimidazole 25

20 grammes d'orthophénylène diamine (0,185 mol.) sont dissouts dans 80 mL d'acétate d'éthyle et placés dans une autoclave équipée d'un manomètre, de deux vannes (entrée et sortie) et d'un disque de rupture, préalablement sous cycles vide/azote. 41,6 grammes de hexafluoropropène (HFP) (0,277 mol.) sont alors introduits à température ambiante dans l'autoclave.

Le milieu est porté à 75°C et la pression dans le réacteur est alors proche de 13 bar. Après 18 heures de réaction, la pression dans le réacteur est voisine des 4 bar, et l'autoclave est refroidie, dégazée à 0°C puis le solvant est évaporé sous pression réduite.

Le solide marron-vert obtenu est repris dans 100 mL d'eau, on le neutralise au moyen d'une solution de Na₂CO₃ (10% en masse) jusqu'à ce que le pH de la solution obtenue soit égal à 7. Le précipité formé est filtré, séché sous vide puis recristallisé dans un mélange H₂O/EtO pour donner 27 g (75%) de l'intermédiaire 25 désiré.

### - Etape (b) : Synthèse du 1-benzyl-2-(1,2,2,2-tetrafluoroéthyl)-benzimidazole 10

10 grammes de 2-(1,2,2,2-tetrafluoroéthyl)-benzimidazole 25 (0,05 mol.) dont la synthèse est décrite ci-dessus et 7,3 grammes de chlorure de benzyle (0,05 mol), sont dissouts dans 140 mL de CH₃CN.

5,2 grammes de K₂CO₃ (0,037 mol) sont alors additionnés et le milieu réactionnel est porté à reflux durant 48 heures.

Le solvant est filtré puis évaporé sous pression réduite.

L'huile noire obtenue est dissoute dans 150 mL de CH₂Cl₂, lavée à l'eau (2 fois 50 mL), séchée sur Na₂SO₄, filtrée puis évaporée sous pression réduite.

Le produit obtenu est à nouveau dissout dans 15 mL d'acétate d'éthyle, précipité dans 200 mL d'heptane, filtrée puis le filtrat est évaporé sous pression réduite.

Les 9 grammes de produit jaune clair recueillies sont recristallisées dans 15 mL d'hexane pour donner 6,3 g (43%) de produit pur 27 (poudre jaune clair).

### - Etape (c) : Synthèse du 1-benzyl-2-(1,2,2-trifluorovinyl)-benzimidazole 21b'

Dans un schlenk de 100 mL préalablement purgé à l'azote, 3 grammes de 1-benzyl-2-(1,2,2,2-tetrafluoroéthyl)-benzimidazole 27 (0,01 mol.) sont dissoutes dans 50 mL de tetrahydrofurane (THF) préalablement séché sur hydrure de calcium puis fraîchement distillé sous argon.

Le système est refroidi à -78°C sous un léger courant d'azote puis 6,5 mL de n-BuLi (1,6 M dans l'hexane, 0,01 mol.) sont additionnés goutte à goutte.

Le milieu réactionnel est ensuite maintenu à basse température durant 1 heure puis laissé sous agitation à température ambiante durant 24 heures.

Enfin, 5 mL d'EtOH sont ajoutés en fin de réaction puis le solvant est évaporé sous pression réduite. Le produit est repris dans 40 mL de CH₂Cl₂, lavé à l'eau (2 fois 20 mL), séché sur Na₂SO₄, puis le solvant est évaporé sous pression réduite.

Les spectres RMN ¹H et RMN ¹⁹F illustrés sur la figure 17 confirment l'obtention du composé souhaité étant donné qu'ils mettent respectivement en évidence la présence des protons du groupement de protection benzyle ainsi que celle des protons de l'hétérocycle benzimidazole (RMN ¹H) et la présence de la double liaison perfluorée de ce monomère étant donné que l'on observe sur le spectre de RMN ¹⁹F, les doublets de doublets de doublets caractéristiques de cette liaison, centrés à -105, - 124 et -175 ppm.

Le monomère 21b' obtenu à l'issu de l'étape (c) peut être homopolymérisé par amorçage radicalaire ou copolymérisé avec des oléfines fluorées telles que le tetrafluoroéthylène, le trifluoroéthylene, le fluorure de vinyle, le 2H-1,1,3,3,3-pentafluoroéthylène, l'hexafluoropropène (HFP), le chlorotrifluoroéthylène (CTFE) ou le fluorure de vinylidène (VDF) afin d'obtenir un polymère protégé qui sera forcément fluoré.

Après l'obtention de ce polymère, il est possible de procéder à la déprotection des atomes d'azote portant le groupement benzyle de protection, par laquelle on obtient un polymère déprotégé dont le motif est constitué par le monomère 21b de la figure 18 nommé 2-(1,2,2-trifluorovinyl)-benzimidazole.

Ce polymère aux atomes d'azote déprotégés, c'est-à-dire porteurs d'un atome d'hydrogène et non plus d'un groupement de protection, est en conséquence conducteur de protons.

On décrit dans ce qui suit la synthèse d'un monomère 21c' semblable au monomère 21b' ci-dessus décrit, mais qui comprend un autre groupement de protection de l'atome d'azote du groupement benzimidazole.

Plus précisément, le monomère 21c' comprend comme élément de protection un composé R pouvant être un groupement trialkylsilyl (R1R2R3Si) ou encore un groupement carbamate (CO₂R4) dans lesquels les groupements R1 à R4 peuvent être constitués par des chaînes alkyles de 1 à 20 atomes de carbone.

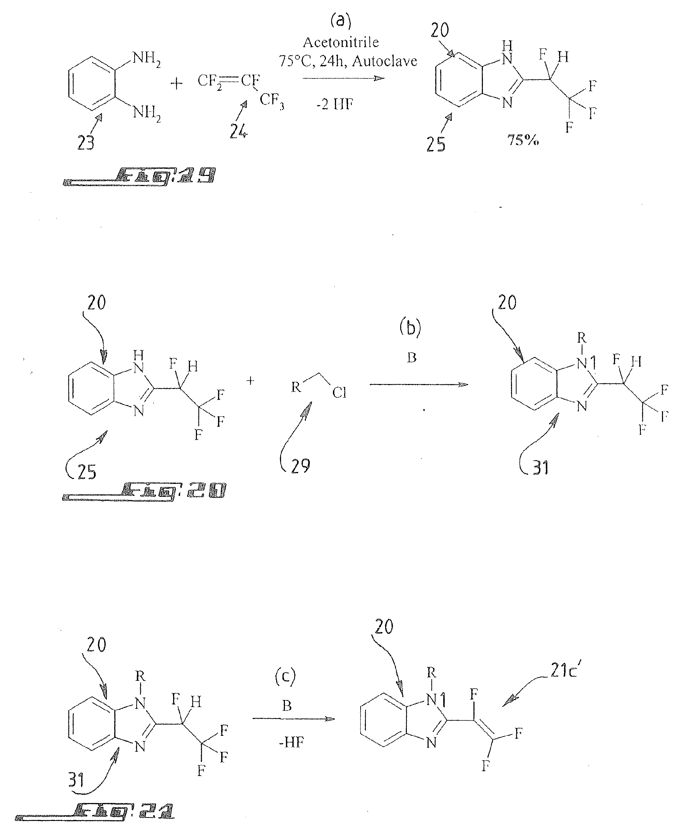

Ce monomère 21c' peut être obtenu par un troisième procédé de fabrication en trois étapes, illustrées sur les figures 19 à 21.

La première étape (a) de la figure 19, connue de l'homme du métier et déjà illustrée sur la figure 15, concerne la réaction entre l'orthophenylènediamine 23 et l'hexafluoropropène 24 à 75°C dans l'acétonitrile, sous autoclave et pendant 24 heures, dont résulte la molécule intermédiaire 25 soit le 2-(1,2,212-tetrafluoroethyl)-benzimidazole.

Le rendement de cette réaction est de 75% après recristallisation dans un mélange H₂O/EtOH.

La seconde étape (b), représentée sur la figure 20, est une réaction de protection de l'un des atomes azotes du groupement benzimidazole 20 de la molécule intermédiaire 25, au moyen d'un réactif 29 de formule générale RCl où R peut être un groupement trialkylsilyl (R1R2R3Si) ou encore un groupement carbamate (CO₂R1).

La réaction est effectuée dans un solvant organique anhydre usuel tel que le tetrahydrofurane (THF), le dioxanne ou le diméthylformamide (DMF) en présence d'une base B telle que le butyl lithium (BuLi), le ter-butyl lithium (t-BuLi), le diisopropylamine de lithium (LDA) ou l'hydrure de sodium (NaH).

On obtient par cette réaction un deuxième composé intermédiaire 31 dont l'azote N1 est protégé par le groupement R.

La troisième étape (c) du procédé, mise en évidence sur la figure 21, est une réaction de déshydrofluoration du composé 31, au moyen d'une base forte telle que le butyl lithium (BuLi), le ter-butyl lithium (t-BuLi), ou la diisopropylamine de lithium (LDA), dans un solvant anhydre tel que le tetrahydrofurane (THF), le dioxanne ou le dimethylformamide (DMF).

Cette réaction permet d'obtenir le monomère 21c' dont les atomes d'azote sont protégés par le groupement R, qui est un groupement trialkylsilyl ou carbamate.

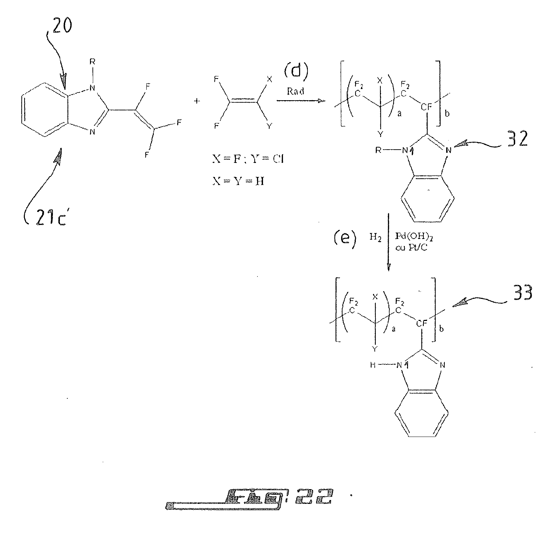

Comme décrit pour le cas du monomère 21b', le monomère 21c' ainsi obtenu peut être homopolymérisé par amorçage radicalaire ou copolymérisé avec des oléfines fluorées tel qu'illustré par l'étape (d) de la figure 22.

Ces oléfines fluorées peuvent être constituées par l'hexafluoropropène (HFP), le chlorotrifluoroéthylène (CTFE) ,le fluorure de vinylidène (VDF) le tetrafluoroéthylène, le trifluoroéthylene, le fluorure de vinyle, ou le 2H-1,1,3,3,3-pentafluoroéthylène.

On obtient par cette étape (d) un polymère fluoré 32 dans lequel les atomes d'azote N1 des hétérocycles sont protégés par un groupement R.

Ce polymère protégé 32 subit ensuite une étape de déprotection au cours de laquelle les groupements R sont remplacés par des atomes d'hydrogène, permettant au polymère déprotégé formé 33 d'être conducteur protonique.

En outre, le polymère déprotégé obtenu présente une inertie chimique en milieu oxydant, du fait de la présence d'atomes de fluor et une thermostabilité, du fait que la conduction protonique est assurée par les hétérocycles ancrés, le prédestinant à la fabrication de membrane utilisée au sein d'une pile à combustible.

Il est à noter que différents monomères peuvent être fabriqués sur la base des étapes (a), (b) et (c) du procédé de fabrication ci-dessus décrit, en remplaçant par exemple l'orthophénylène diamine 23 de l'étape (a) par un composé comprenant une telle orthophénylène diamine 23.

De même, d'autres polymères perhalogénés, fluorés ou partiellement fluorés peuvent être formés à partir de monomères perhalogénés, fluorés ou partiellement fluorés et porteurs de groupements benzimidazole, puis déprotégés pour former un polymère conducteur protonique.

Par conséquent, plusieurs types de membranes conductrices ioniques peuvent être fabriquées à partir des polymères déprotégés perhalogénés, pour constituer différents types d'électrolyte qui pourront être intégrés au sein de nombreux dispositifs électrochimiques, tels qu'un électrolyseur, une pile à combustion.

Les membranes selon l'invention permettent notamment de dépasser la température limite de fonctionnement des piles à combustible à électrolyte solide polymère connues, de réduire la complexité du Système Pile (pile et ses auxiliaires) étant donné que la pile selon l'invention est anhydre, et donc par conséquent son coût (les auxiliaires dédiés à humidification n'étant plus nécessaires) et présente une durée de vie plus longue que les piles usuelles.

Les hétérocycles des polymères formés suivant les procédés de fabrication précités, assurent le transport protonique au sein de la membrane, alors que les groupements fluorés présents au sein du polymère dotent le polymère d'une certaine inertie chimique en milieu oxydant ou réducteur et d'une thermostabilité qui participent à la stabilisation de la membrane en fonctionnement, notamment pour une utilisation au sein d'une pile à combustible opérant à des températures supérieures à 100°C.

## Revendications

1. Monomère porteur d'un hétérocycle de type imidazole, pouvant être utilisé pour la fabrication d'une membrane conductrice ionique, **caractérisé en ce que** sa structure chimique comprend au moins un motif de formule : 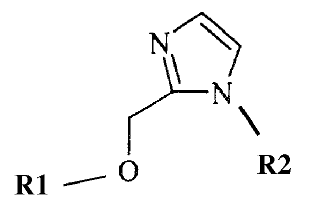 dans laquelle R1 comprend un groupement alkenyl, et dans laquelle R2 comprend un groupement de protection de l'un des atomes d'azote de l'hétérocycle choisi parmi un groupement benzyle ou trialkylsilyl.

2. Monomère selon la revendication 1, dans lequel R1 est de formule chimique CH=CH₂.

3. Monomère selon la revendication 1, dans lequel R1 est de formule chimique (CH₂)₂-O-CH=CH₂.

4. Procédé de fabrication du monomère de l'une quelconque des revendications 1 à 3, **caractérisé en ce qu'**il comprend au moins une étape de formation (a) du 1-benzyl-2-(hydroxyméthyl)imidazole (7) et au moins une étape de synthèse dudit monomère (6, 14) à partir de ce 1-benzyl-2-(hydroxyméthyl)imidazole (7).

5. Procédé selon la revendication 4, dans lequel l'étape de synthèse du monomère (14) est une transétherification du 1-benzyl-2-(hydroxyméthyl)imidazole (7) par l'éthyl vinyl éther (15), ce dont il résulte le 1-benzyl-2-(vinyloxyméthyl)imidazole (14).

6. Procédé selon la revendication 5, dans lequel la transétherification est effectuée en présence d'acétate de mercure (16).

7. Procédé selon la revendication 4, dans lequel l'étape de synthèse du monomère (6) consiste en une réaction entre le 1-benzyl-2-(hydroxyméthyl)imidazole (7) et le chloroéthyl vinyl éther (8) en présence d'un catalyseur par transfert de phase, ce dont il résulte le 1-benzyl-2-(vinyloxyéthyloxyméthyl)imidazole (6).

8. Procédé selon la revendication 7, dans lequel le catalyseur de transfert de phase est le chlorure de triéthyl benzyl ammonium.

9. Polymère protégé comprenant le monomère de l'une quelconque des revendications 1 à 3, ou comprenant le monomère fabriqué par l'un quelconque des procédés des revendications 4 à 7.

10. Polymère protégé selon la revendication 9, comprenant en outre des fonctions acides.

11. Procédé de fabrication du polymère protégé de la revendication 9 ou 10, à partir du monomère de l'une quelconque des revendications 1 à 3, comprenant au moins une étape de polymérisation de ce monomère (6, 14).

12. Procédé selon la revendication 11, dans lequel l'étape de polymérisation consiste en une homopolymérisation cationique du monomère (6, 14) en présence d'un amorceur cationique, tel que du ZnI₂, HgI₂ ou des sels de Crivello.

13. Procédé selon la revendication 11, dans lequel l'étape de polymérisation consiste en une copolymérisation radicalaire de ce monomère avec un second monomère.

14. Procédé selon la revendication 11, dans lequel l'étape de polymérisation consiste en une terpolymérisation du monomère (6, 14) avec deux seconds monomères.

15. Procédé selon la revendication 13 ou 14, dans lequel le second monomère est un monomère vinylique accepteur d'électrons, tel qu'un monomère fluoré ou perfluoré (11).

16. Procédé selon la revendication 15, **caractérisé en ce que** le monomère fluoré (11) est choisi parmi la famille des alcènes fluorés, tels que le chlorotrifluoroéthylène, l'hexafluoropropène, le trifluoroéthylène, le tetrafluoroéthylène, le 3,3,3-trifluoropropene, le pentafluoropropène, des oléfines perfluorées, et/ou des fluorures de vinylidène, perfluoroalkyl vinyl éther, perfluoroalkoxyalkyl vinyl éther un perfluoroalkylvinyléther.

17. Polymère déprotégé (12') fait à base du polymère protégé (12) des revendications 9 ou 10 ou à base du polymère fabriqué à partir de l'un quelconque des procédés des revendications 11 à 16, comprenant des atomes d'hydrogène en remplacement des groupements de protection (R2) du polymère protégé (12).

18. Procédé de fabrication du polymère protégé de la revendication 17, utilisant le polymère protégé (12) de la revendication 9 ou 10 ou le polymère fabriqué à partir de l'un quelconque des procédés des revendications 11 à 16, **caractérisé en ce qu'**il comprend une étape de déprotection des atomes d'azote des hétérocycles du polymère protégé, 12 par laquelle les groupements de protection (R2) du polymère (12) sont remplacés par des atomes d'hydrogène, formant un polymère déprotégé (12') pourvu d'hétérocycles de type imidazole.

19. Procédé selon la revendication 17, **caractérisé en ce que** l'étape de déprotection consiste en une réaction du polymère protégé (12) obtenu par l'étape de polymérisation avec le Pd(OH)2.

20. Monomère porteur d'un hétérocycle de type benzimidazole, pouvant être utilisé pour la fabrication d'une membrane conductrice ionique, **caractérisé en ce que** sa structure chimique comprend au moins un motif de formule : 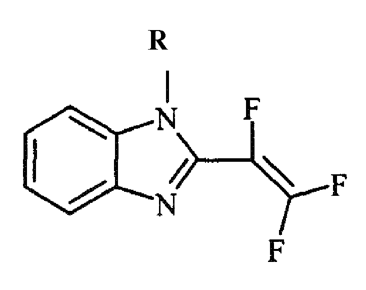 dans laquelle R est un groupement benzyl ou trialkylsilyl de protection de l'un des atomes d'azote de l'hétérocycle.

21. Procédé de fabrication du monomère de la revendication 20, **caractérisé en ce qu'**il comprend :
- au moins une étape de réaction (a) entre un composé comprenant une ortho-phénylène diamine, tel qu'une orthophénylène diamine, et un monomère fluoré, tel que l'hexafluoropropène (HFP), en présence d'un solvant organique tel que l'acétonitrile, par laquelle est obtenue une molécule intermédiaire (10) fluorée et porteuse d'un hétérocycle de type benzimidazole (5),
- au moins une étape de protection (b) d'un atome d'azote (N1) du groupement benzimidazole (5) de la molécule intermédiaire (10) par un groupement R choisi parmi un groupement benzyl ou un groupement trialkylsilyl (R1R2R3Si),
- au moins une étape de déshydrofluoration (c) du composé obtenu à l'étape (b) par réaction de ce composé avec une base, par laquelle est formé un monomère protégé (6b', 6c').

22. Procédé selon la revendication 21, dans lequel l'étape de synthèse (a) est effectuée à une température telle que 75°C.

23. Procédé selon la revendication 22, dans lequel l'étape de synthèse (a) est réalisée dans l'autoclave sous une haute pression, telle que 5-50 bar.

24. Procédé selon l'une des revendications 21 à 23, dans lequel l'étape de protection (b) est une réaction entre la molécule intermédiaire (10) et un réactif de formule R-X, tel que le chlorure de benzyle Ph-Cl, réalisée en présence d'une base.

25. Procédé selon la revendication 21 ou 24, dans lequel les bases utilisées dans les étapes (b) et (c) sont choisies parmi le butyl lithium, le ter-butyl lithium, le diisopropylamine de lithium ou l'hydrure de sodium.

26. Procédé selon l'une des revendications 21 à 25, dans lequel les étapes (b) et (c) sont conduites en présence d'un solvant organique anhydre tel que le tetrahydrofurane (THF), le dioxanne ou le diméthylformamide (DMF).

27. Procédé selon l'une des revendications 21 à 26, dans lequel l'étape de déhydrofluoration (c) est effectuée au moins en partie à basse température, telle que -78°C.

28. Polymère protégé (17) comprenant le monomère de la revendication 20.

29. Polymère protégé (17) selon la revendication 28, comprenant en outre des fonctions acides.

30. Polymère déprotégé fait à base du polymère des revendications 28 ou 29, comprenant des atomes d'hydrogène en remplacement des groupements de protection (R).

31. Membrane conductrice ionique, faite à base du polymère déprotégé de la revendication 17 ou du polymère fabriqué selon l'un quelconque des procédés des revendications 18 à 19 ou du polymère déprotégé de la revendication 30.

32. Dispositif électrochimique impliquant comme électrolyte une membrane conductrice ionique de la revendication 31.

33. Pile à combustible impliquant comme électrolyte la membrane conductrice ionique de la revendication 32.

34. Utilisation de la pile à combustible de la revendication 33 pour assurer la traction d'un véhicule automobile.

## Patentansprüche

1. Monomer, das einen Heterocyclus vom Imidazoltyp trägt, das für die Herstellung einer ionenleitenden Membran verwendet werden kann, **dadurch gekennzeichnet, dass** seine chemische Struktur mindestens ein Motiv der folgenden Formel umfasst: 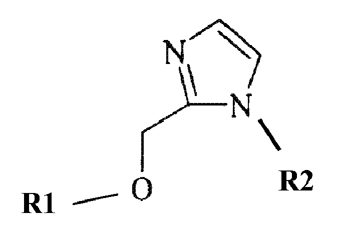 worin R1 eine Alkenylgruppe umfasst und worin R2 eine Schutzgruppe für eines der Stickstoffatome des Heterocyclus umfasst, wie etwa eine Benzyl- oder Trialkylsilylgruppe.

2. Monomer nach Anspruch 1, wobei R1 die chemische Formel CH=CH₂ ist.

3. Monomer nach Anspruch 1, wobei R1 die chemische Formel (CH₂)₂-O-CH=CH₂ ist.

4. Verfahren zur Herstellung des Monomers nach einem der Ansprüche 1 bis 3, **dadurch gekennzeichnet, dass** es mindestens einen Schritt zur Bildung (a) von 1-Benzyl-2-(hydroxymethyl)imidazol (7) und mindestens einen Schritt zur Synthese des Monomers (6, 14) aus diesem 1-Benzyl-2-(hydroxymethyl)imidazol (7) umfasst.

5. Verfahren nach Anspruch 4, wobei es sich bei dem Schritt zur Synthese des Monomers (14) um eine Umetherung von 1-Benzyl-2-(hydroxymethyl)imidazol (7) durch Ethylvinylether (15) handelt, woraus 1-Benzyl-2-(vinyloxymethyl)imidazol (14) resultiert.

6. Verfahren nach Anspruch 5, wobei die Umetherung in Gegenwart von Quecksilberacetat (16) durchgeführt wird.

7. Verfahren nach Anspruch 4, wobei der Schritt zur Synthese des Monomers (6) aus einer Reaktion zwischen 1-Benzyl-2-(hydroxymethyl)imidazol (7) und dem Chlorethylvinylether (8) in Gegenwart eines Phasentransferkatalysators besteht, woraus 1-Benzyl-2-(vinyloxyethyloxymethyl)imidazol (6) resultiert.

8. Verfahren nach Anspruch 7, wobei es sich bei dem Phasentransferkatalysator um Triethylbenzylammoniumchlorid handelt.

9. Geschütztes Polymer, umfassend das Monomer nach einem der Ansprüche 1 bis 3 oder umfassend das Monomer, hergestellt mit einem der Verfahren der Ansprüche 4 bis 7.

10. Geschütztes Polymer nach Anspruch 9, ferner umfassend Säurefunktionen.

11. Verfahren zur Herstellung des geschützten Polymers des Anspruchs 9 oder 10 aus dem Monomer nach einem der Ansprüche 1 bis 3, umfassend mindestens einen Schritt zur Polymerisation dieses Monomers (6, 14).

12. Verfahren nach Anspruch 11, wobei der Schritt zur Polymerisation aus einer kationischen Homopolymerisation des Monomers (6, 14) in Gegenwart eines kationischen Initiators, wie etwa ZnI₂, HgI₂ oder Crivello-Salzen, besteht.

13. Verfahren nach Anspruch 11, wobei der Schritt zur Polymerisation aus einer radikalischen Copolymerisation dieses Monomers mit einem zweiten Monomer besteht.

14. Verfahren nach Anspruch 11, wobei der Schritt zur Polymerisation aus einer Terpolymerisation des Monomers (6, 14) mit zwei zweiten Monomeren besteht.

15. Verfahren nach Anspruch 13 oder 14, wobei es sich bei dem zweiten Monomer um ein elektronenakzeptierendes Vinylmonomer, wie etwa ein fluoriertes oder perfluoriertes Monomer (11), handelt.

16. Verfahren nach Anspruch 15, **dadurch gekennzeichnet, dass** das fluorierte Monomer (11) ausgewählt ist aus der Familie der fluorierten Alkene, wie etwa Chlortrifluorethylen, Hexafluorpropen, Trifluorethylen, Tetrafluorethylen, 3,3,3-Trifluorpropen, Pentafluorpropen, perfluorierten Olefinen und/oder Vinylidenfluoriden, Perfluoralkylvinylether, Perfluoralkoxyalkylvinylether Perfluoralkylvinylether.

17. Entschütztes Polymer (12'), hergestellt auf der Basis des geschützten Polymers (12) der Ansprüche 9 oder 10 oder auf der Basis des Polymers, hergestellt nach einem der Verfahren der Ansprüche 11 bis 16, umfassend Wasserstoffatome, die die Schutzgruppen (R2) des geschützten Polymers (12) ersetzen.

18. Verfahren zur Herstellung des geschützten Polymers des Anspruchs 17 unter Verwendung des geschützten Polymers (12) des Anspruchs 9 oder 10 oder des Polymers, hergestellt nach einem der Verfahren der Ansprüche 11 bis 16, **dadurch gekennzeichnet, dass** es einen Schritt zur Entschützung der Stickstoffatome der Heterocyclen des geschützten Polymers 12 umfasst, wodurch die Schutzgruppen (R2) des Polymers (12) durch Wasserstoffatome ersetzt werden, die ein entschütztes Polymer (12') bilden, das frei von Heterocyclen vom Imidazoltyp ist.

19. Verfahren nach Anspruch 17, **dadurch gekennzeichnet, dass** der Schritt zur Entschützung in einer Umsetzung des geschützten Polymers (12), das durch den Schritt zur Polymerisation erhalten wurde, mit Pd(OH)₂ besteht.

20. Monomer, das einen Heterocyclus vom Benzimidazoltyp trägt, das für die Herstellung einer ionenleitenden Membran verwendet werden kann, **dadurch gekennzeichnet, dass** seine chemische Struktur mindestens eine Einheit der folgenden Formel umfasst: 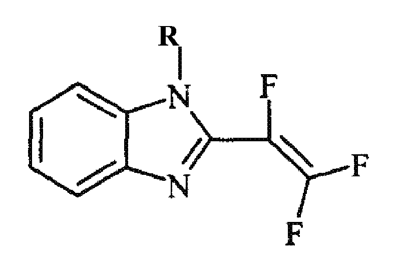 worin R eine Schutzgruppe eines der Stickstoffatome des Heterocyclus ist.

21. Verfahren zur Herstellung des Monomers aus Anspruch 20, **dadurch gekennzeichnet, dass** es umfasst:
- mindestens einen Schritt zur Umsetzung (a) einer Verbindung, umfassend ein ortho-Phenylendiamin, wie etwa ein ortho-Phenylendiamin, und eines fluorierten Monomers, wie etwa Hexafluorpropen (HFP), in Gegenwart eines organischen Lösemittels, wie etwa Acetonitril, wodurch ein Zwischenmolekül (10) erhalten wird, das fluoriert ist und einen Heterocyclus vom Benzimidazoltyp (5) trägt,
- mindestens einen Schritt zum Schützen (b) eines Stickstoffatoms (N1) der Benzimidazolgruppe (5) des Zwischenmoleküls (10) durch eine Gruppe R, ausgewählt aus einer Benzylgruppe oder einer Trialkylsilylgruppe (R1R2R3Si),
- mindestens einen Schritt zur Dehydrofluorierung (c) der in Schritt (b) erhaltenen Verbindung durch Umsetzung dieser Verbindung mit einer Base, wodurch ein geschütztes Monomer (6b', 6c') gebildet wird.

22. Verfahren nach Anspruch 21, wobei der Schritt zur Synthese (a) bei einer Temperatur, wie etwa 75 °C, durchgeführt wird.

23. Verfahren nach Anspruch 22, wobei der Schritt zur Synthese (a) im Autoklaven unter hohem Druck, wie etwa 5 - 50 bar, durchgeführt wird.

24. Verfahren nach einem der Ansprüche 21 bis 23, wobei es sich bei dem Schritt zum Schützen (b) um eine Umsetzung des Zwischenmoleküls (10) mit einem Reagens der Formel R-X, wie etwa Benzylchlorid Ph-Cl handelt, die in Gegenwart einer Base durchgeführt wird.

25. Verfahren nach Anspruch 21 oder 24, wobei die in den Schritten (b) und (c) verwendeten Basen ausgewählt sind aus Butyllithium, tert-Butyllithium, Lithiumdiisopropylamin oder Natriumhydrid.

26. Verfahren nach einem der Ansprüche 21 bis 25, wobei die Schritte (b) und (c) in Gegenwart eines wasserfreien, organischen Lösemittels, wie etwa Tetrahydrofuran (THF), Dioxan oder Dimethylformamid (DMF), durchgeführt werden.

27. Verfahren nach einem der Ansprüche 21 bis 26, wobei der Schritt zur Dehydrofluorierung (c) mindestens teilweise bei niedriger Temperatur, wie etwa -78 °C, durchgeführt wird.

28. Geschütztes Polymer (17), umfassend das Monomer aus Anspruch 20.

29. Geschütztes Polymer (17) nach Anspruch 28, ferner umfassend Säurefunktionen.

30. Entschützes Polymer, hergestellt auf der Basis des Polymers der Ansprüche 28 oder 29, umfassend Wasserstoffatome, die Schutzgruppen (R) ersetzen.

31. Ionenleitende Membran, hergestellt auf der Basis des Polymers aus Anspruch 17 oder des Polymers, hergestellt nach einem der Verfahren der Ansprüche 18 bis 19 oder des entschützten Polymers aus Anspruch 30.

32. Elektrochemische Vorrichtung, die als Elektrolyten eine ionenleitende Membran nach Anspruch 31 umfasst.

33. Brennstoffzelle, die als Elektrolyten die ionenleitende Membran nach Anspruch 32 umfasst.

34. Verwendung der Brennstoffzelle nach Anspruch 33 um den Antrieb eines Kraftfahrzeugs zu gewährleisten.

## Claims

1. A monomer carrying a heterocycle of the imidazole type, capable of being used for the production of an ionic conductive membrane, **characterized in that** its chemical structure comprises at least one unit of the formula: 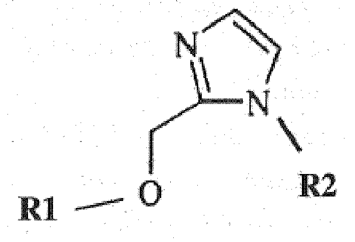 in which R1 comprises an alkenyl group, and in which R2 comprises a protection group of one of the nitrogen atoms of the heterocycle, selected from a benzyl or trialkylsilyl group.

2. The monomer according to claim 1, in which R1 has the chemical formula CH=CH₂.

3. The monomer according to claim 1, in which R1 has the chemical formula (CH₂)₂-O-CH=CH₂.

4. A method for the production of the monomer of any one of claims 1 to 3, **characterized in that** it comprises at least one step (a) of forming 1-benzyl-2-(hydroxymethyl)imidazole (7) and at least one step of synthesizing the said monomer (6, 14) from this 1-benzyl-2-(hydroxymethyl)imidazole (7).

5. The method according to claim 4, in which the step of synthesizing the monomer (14) is a transetherification of the 1-benzyl-2-(hydroxymethyl)imidazole (7) by ethyl vinyl ether (15), from which 1-benzyl-2-(vinyloxymethyl)imidazole (14) results.

6. The method according to claim 5, in which the transetherification is carried out in the presence of mercury acetate (16).

7. The method according to claim 4, in which the step of synthesizing the monomer (6) consists of a reaction between the 1-benzyl-2-(hydroxymethyl)imidazole (7) and chloroethyl vinyl ether (8) in the presence of a phase transfer catalyst, from which 1-benzyl-2-(vinyloxyethyloxymethyl)imidazole (6) results.

8. The method according to claim 7, in which the phase transfer catalyst is triethyl benzyl ammonium chloride.

9. A protected polymer comprising the monomer of any one of claims 1 to 3, or comprising the monomer produced by any one of the methods of claims 4 to 7.

10. The protected polymer according to claim 9, further comprising acid functions.

11. A method for the production of the protected polymer of claim 9 or 10, from the monomer of any one of claims 1 to 3, comprising at least one polymerisation step of this monomer (6, 14).

12. The method according to claim 11, in which the polymerisation step consists of a cationic homopolymerisation of the monomer (6, 14) in the presence of a cationic initiator, such as ZnI₂, HgI₂ or Crivello salts.

13. The method according to claim 11, in which the polymerisation step consists of a radical copolymerisation of this monomer with a second monomer.

14. The method according to claim 11, in which the polymerisation step consists of a terpolymerisation of the monomer (6, 14) with two second monomers.

15. The method according to claim 13 or 14, in which the second monomer is an electron-accepting vinyl monomer, such as a fluorinated or perfluorinated monomer (11).

16. The method according to claim 15, **characterized in that** the fluorinated monomer (11) is selected from the family of fluorinated alkenes, such as chlorotrifluoroethylene, hexafluoropropene, trifluoroethylene, tetrafluoroethylene, 3,3,3-trifluoropropene, pentafluoropropene, perfluorinated olefins, and/or vinylidene fluorides, perfluoroalkyl vinyl ether, perfluoro alkoxyalkyl vinyl ether a perfluoro alkyl vinyl ether.

17. A deprotected polymer (12') made based on the protected polymer (12) of claims 9 or 10 or based on the polymer produced from any one of the methods of claims 11 to 16, comprising hydrogen atoms replacing the protection groups (R2) of the protected polymer (12).

18. A method for the production of the protected polymer of claim 17, using the protected polymer (12) of claim 9 or 10 or the polymer produced from any one of the methods of claims 11 to 16, **characterized in that** it comprises a step of deprotection of the nitrogen atoms of the heterocycles of the protected polymer 12, by which the protection groups (R2) of the polymer (12) are replaced by hydrogen atoms, forming a deprotected polymer (12') provided with heterocycles of the imidazole type.

19. The method according to claim 17, **characterized in that** the deprotection step consists of a reaction of the protected polymer (12) obtained by the polymerisation step with Pd(OH)2.

20. A monomer carrying a heterocycle of the benzimidazole type, being able to be used for the production of an ionic conductive membrane, **characterized in that** its chemical structure comprises at least one unit of the formula: 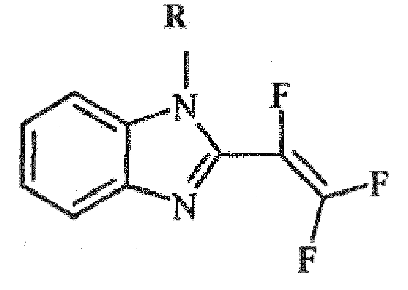 in which R is a benzyl or trialkylsilyl protection group of one of the nitrogen atoms of the heterocycle.

21. A method for the production of the monomer of claim 20, **characterized in that** it comprises:
- at least one reaction step (a) between a compound comprising an ortho-phenylene diamine, such as an ortho-phenylene diamine, and a fluorinated monomer, such as hexafluoropropene (HFP), in the presence of an organic solvent such as acetonitrile, by which a fluorinated intermediate molecule (10) carrying a heterocycle of the benzimidazole type (5) is obtained,
- at least one step (b) of protecting a nitrogen atom (N1) of the benzimidazole group (5) of the intermediate molecule (10) by a group R selected from a benzyl group or a trialkylsilyl group (R1R2R3Si),
- at least one step (c) of dehydrofluorinating of the compound obtained at step (b) by reaction of this compound with a base, by which a protected monomer (6b', 6c') is formed.

22. The method according to claim 21, in which the synthesis step (a) is carried out at a temperature such as 75 °C.

23. The method according to claim 22, in which the synthesis step (a) is carried out in the autoclave under high pressure, such as 5 - 50 bar.

24. The method according to one of claims 21 to 23, in which the protection step (b) is a reaction between the intermediate molecule (10) and a reagent of the formula R-X, such as benzyl chloride Ph-Cl, carried out in the presence of a base.

25. The method according to claim 21 or 24, in which the bases used in steps (b) and (c) are selected from butyl lithium, ter-butyl lithium, lithium diisopropylamine or sodium hydride.

26. The method according to one of claims 21 to 25, in which the steps (b) and (c) are carried out in the presence of an anhydrous organic solvent such as tetrahydrofurane (THF), dioxane or dimethylformamide (DMF).

27. The method according to one of claims 21 to 26, in which the dehydrofluorination step (c) is carried out at least in part at low temperature, such as -78 °C.

28. A protected polymer (17) comprising the monomer of claim 20.

29. The protected polymer (17) according to claim 28, further comprising acid functions.

30. A deprotected polymer made based on the polymer of claims 28 or 29, comprising hydrogen atoms replacing protection groups (R).

31. An ionic conductive membrane made based on the deprotected polymer of claim 17 or on the polymer produced according to any one of the methods of claims 18 to 19 or on the deprotected polymer of claim 30.

32. An electrochemical device involving as electrolyte an ionic conductive membrane of claim 31.

33. A fuel cell involving as electrolyte the ionic conductive membrane of claim 32.

34. The use of the fuel cell of claim 33 to provide the traction of a motor vehicle.
